# EUROPEAN PATENT SPECIFICATION

(11) **EP 2 833 793 B1**
(45) Date of publication and mention of the grant of the patent: **12.07.2017**
(21) Application number: 12872591.8
(22) Date of filing: 30.03.2012
(51) Int. Cl.: A61B 10/00, A61F 13/84, G01N 33/493, A61F 5/451, C12M 1/28, A61F 17/00

(54) **URINE SAMPLING DEVICE**
VORRICHTUNG ZUR URINPROBENAHME
DISPOSITIF DE COLLECTE D'URINE

(43) Date of publication of application: 11.02.2015
(73) Proprietor: SCA HYGIENE PRODUCTS AB, 405 03 Göteborg (SE)
(72) Inventor: HUSMARK, Ulrika, S-435 38 Mölnlycke (SE); GUSTAFSON, Ingrid, S-430 31 Åsa (SE)
(74) Representative: Zacco Sweden AB
(86) International application number: PCT/SE2012/050358
(87) International publication number: WO 2013/147661

(56) References cited:
- WO-A1-00/39579
- WO-A1-97/43988
- WO-A1-2004/021890
- WO-A2-00/49948
- WO-A2-03/009798
- WO-A2-2004/030721
- WO-A2-2008/131904
- DE-U1-202004 008 954
- GB-A- 2 131 298
- US-A- 3 918 433
- US-A1- 2003 208 170
- US-A1- 2004 133 090
- US-A1- 2006 062 854
- US-A1- 2008 269 706
- US-A1- 2008 274 014
- US-A1- 2011 145 993
- US-A1- 2012 231 060

## Description

The present invention relates to a urine sampling device according to the preamble of claim 1. It also relates to a hygiene absorbent product comprising the urine sampling device and a urine examination kit.

### BACKGROUND

A document in the technical area of the invention is SE 532682 C2, where a diaper is described for collection of urine samples for diagnosing e.g. urine tract infections. The urine from the user is guided through a feeding channel to a urine container located on the outside of the diaper. The urine container is dismountable.

Another document is published under number GB 2 131 298 A and describes a urine specimen collection device comprising a container and a funnel. The device includes a closure associated with the funnel or the container or both.

A further document is published under US 3,918,433 A and desicribes a fluid sampling device for the collection of fluids for examination, the device may be used in combination with a diaper.

A yet further document is published under number US 2008/0274014 A1 and describes a a diagonstic device for testing hydration and other conditions.

WO 03/009798 discloses a portable biological sample device, comprising a housing, a sample chamber for receiving at least a part of an absorbent pad.

US 2011/145993 A1 discloses a fluid disposal system for collecting fluid.

DE 20 2004 008954 U1 discloses a moisture alarm device to be associated with a nappy.

### SHORT DESCRIPTION OF THE INVENTION

The present invention relates to sampling of urine from patients for later bacteria cultivation and analysis thereof. In particular, this invention assists with sampling from individuals where urination cannot be performed on command, such as elderly dement people or small children.

At least one of the problems is solved by the present invention by a urine sampling device comprising: a housing comprising a urine sampling chamber, the housing of the urine sampling device being arranged such that the urine sampling device can be associated with a hygiene absorbent product, and the urine sampling device comprises an inlet for said fluid, wherein an outer surface of the device comprises an attachment means, for attaching the urine sampling device to the body side of the hygiene absorbent product, or the hygiene absorbent product comprises a pocket for incorporating the urine sampling device , and said chamber comprises a dry gel that can be swelled by urine, wherein said gel is a physically cross linked hydrophilic gel, and is chosen from a group consisting of polysaccharides and synthetic hydrophilic polymers, and said hydrophilic polymer is polyethyleneoxide (PEO)and wherein said urine sampling device posses the ability to preserve a bacteria content in the urine sampled in the urine sampling device, either by means of the properties of the dry gel in itself and/or by providing a preservative chosen from a group consisting of acids such as boric acid, and salts from these such as sodium borate or potassium borate, in the dry gel.

The effect of the above invention is that the sampling can be performed without any surveillance of a paramedic. If it is desired to conduct the sampling at night, the urine sampling device can be added to the absorbent product and applied to the individual who is to be tested. The preservation of the bacteria makes it possible to leave the product on all night and in the morning the sampling device can be removed and the analysis conducted. This solves the problem of not being able to communicate with the individual from whom urine is to be sampled in that they do not need to signal when they have urinated and the sampling device can be left all night without the result of the urine sample will give a misleading result.

In accordance with the present invention, said gel is a physically cross linked hydrophilic gel.

Gels of this type have good liquid retaining properties and are suitable for the aimed purpose of urine sampling.

In accordance with the present invention, the gel is chosen from a group consisting of polysaccharides and synthetic polymers, preferably hydrophilic polymers.

The advantage of polysaccharides is that they come from a renewable source, which is considered beneficial for the environment, not least by the consumers. The advantage of synthetic polymers is that they are homogeneous in composition from batch to batch.

According to the said embodiment of said urine sampling device, said hydrophilic polymer is polyethyleneoxide (PEO).

The PEO polymer is particularly advantageous as it provides an inherent preservative effect of its own.

In accordance with the present invention, said preservative is a composition that has acidic and/or desiccant properties.

In accordance with the present invention, the preservative is chosen from a group consisting of acids such as boric acid and salts from these such as sodium borate or potassium borate.

This group of compounds has a proven preservative effect on bacteria causing urinary tract infection.

In accordance with a further development of the present invention said preservative is present in an amount such that the number of bacteria trapped in the chamber should not change during at least 12 hours, The preservative should prevent growth and still not kill the bacteria. The preservative is good and precise enough for the intended use if it secures that growth is less than one log unit and/or death is less than one log unit.

There is an advantage if the preservative allows the sample to be held for a time period that will allow sampling for example over night, in particular for persons that cannot perform a sampling on command.

In accordance with the present invention an outer surface of the urine sampling device comprises an attachment means, for attaching the urine sampling device to the body side of an absorbent product.

It is particularly advantageous to have a urine sampling device that can be attached easily to a standard hygiene absorbent product, without modifying it.

In accordance with a further development of the present invention, said attachment means is arranged such that it can detachably attach the urine sampling device to said absorbent product.

This is particularly advantageous if the sampling person is not the same as the person that performs the analysis of the sampled urine. Then the urine sampling device can be detached for analysis in another location.

According to the said embodiment of the urine sampling device said attachment means is an adhesive, or a hook and loop arrangement.

These attachment means are particularly convenient as they are generally well recognized as having a good function together with hygiene absorbent products in general.

In accordance with a further development of the present invention said chamber comprises a selected amount of dry gel, in order to swell and hold a predetermined volume of urine in the chamber.

By sampling a predetermined volume of urine the found bacteria can directly be counted and consistent analysing results is provided.

According to the said embodiment of the urine sampling device said volume is comprised in the interval 100 µl to 5000 µl preferably 150 µl to 500 µl.

These volume intervals have been found to be appropriate when examining and analysing urine.

In accordance with a further development of the present invention, it comprises a closing device for closing said inlet to seal the chamber when said urine sampling device has received a predetermined volume of urine.

The advantage of providing a closing device is that the sampled urine is prevented from exiting once sampled, at least in any significant amount. Another advantage is that the urine sample stays in place when the urine sampling device is handled after sampling.

In accordance with a further development of the present invention, the urine sampling device is dismountable by means of fractural impressions made in the housing, such that a urine sample absorbed by said gel can be removed from said chamber, for further examination.

The advantage of this is that the urine sample can be easier to extract from the device.

In accordance with a further development of the present invention, the housing of the urine sampling device comprises a ventilation opening for ventilating air out of the urine sampling device when it is filled with urine.

The ventilation opening provides for a better filling of the device, as exiting air need not exit by the same opening as the urine entering.

The invention also comprises a hygiene absorbent product that comprises an associated urine sampling device according to what is mentioned above..

In some sampling situations it is preferred to provide a complete absorbent product when sampling the urine. It is then an advantage if the sampling person or the person to be investigated for urinary tract infections need not handle the device himself. And further the position of the device in the hygiene absorbent product is predetermined and thus sampling can be more consistent, than if the device is positioned in a position of choice.

The invention further comprises a urine examination kit, comprising a urine sampling device according the above and a sample collection container arranged to house the urine sampling device, for transport of said urine sampling device for further examination.

The urine examination kit is of advantage if sampling is performed far from a laboratory making the analysis. The sample in the urine sampling device can then be sent to the analyse laboratory.

The urine examination kit can further comprise a glove for preventing contamination of the urine sampling device when removing it from the hygiene absorbent product.

By providing a glove the handling of the urine sampling device is further improved in terms of contamination, risks.

### SHORT DESCRIPTION OF THE DRAWINGS

Fig. 1 discloses a urine sampling device according to the invention,
Fig. 2 discloses the urine sampling device of Fig. 1 from another angle,
Fig. 3 discloses a hygiene absorbent product according to the invention,
Fig. 4 discloses a kit for sampling urine according to the invention,
Fig. 4a discloses a kit for sampling urine according to the invention,
Fig. 5 discloses a closing device of the inlet of a urine sampling device of the invention,
Fig. 5a discloses an alternative closing device of the inlet of a urine sampling device of the invention.
Fig. 6 discloses a urine sampling device of the invention having fractural impressions,
Fig. 7 discloses a hygiene absorbent product comprising the urine sampling device of the invention.
Fig. 8 Chart for example 1
Fig. 9 First Chart for example 2
Fig. 10 Second Chart for example 2

### DETAILED DESCRIPTION

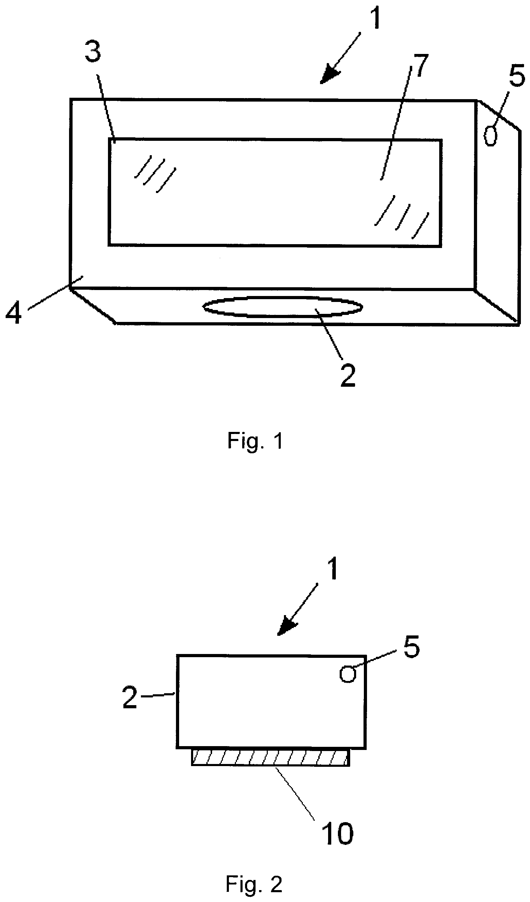

In Fig. 1 a first embodiment of a urine sampling device 1 according to the invention is disclosed. The urine sampling device 1 has a housing 4 comprising a chamber 3. The housing 4 is made in any suitable material. The housing 3 is preferably formed by a sheet of plastic or other suitable material such as paperboard made hydrophobic. The shape of the embodiment of Fig. 1 is a box. The shape of the urine sampling device 1 need not be a box. It can also have a cylinder shape and any other suitable three dimensional shapes. It can also be formed as a flat urine sampling device comprising the dry gel, wherein the urine sampling device can expand together with the gel. The chamber 3 can be of the size ranging from 100-5000 µl. The volume can be altered if needed for analytical reasons, for example 100 µl is thinkable as well as 200 µl or 400 µl. If the volume is too small the necessary amount of bacteria for a good analysis will not be present. A too large volume will make the device too large such that it will cause discomfort to the user during sampling.

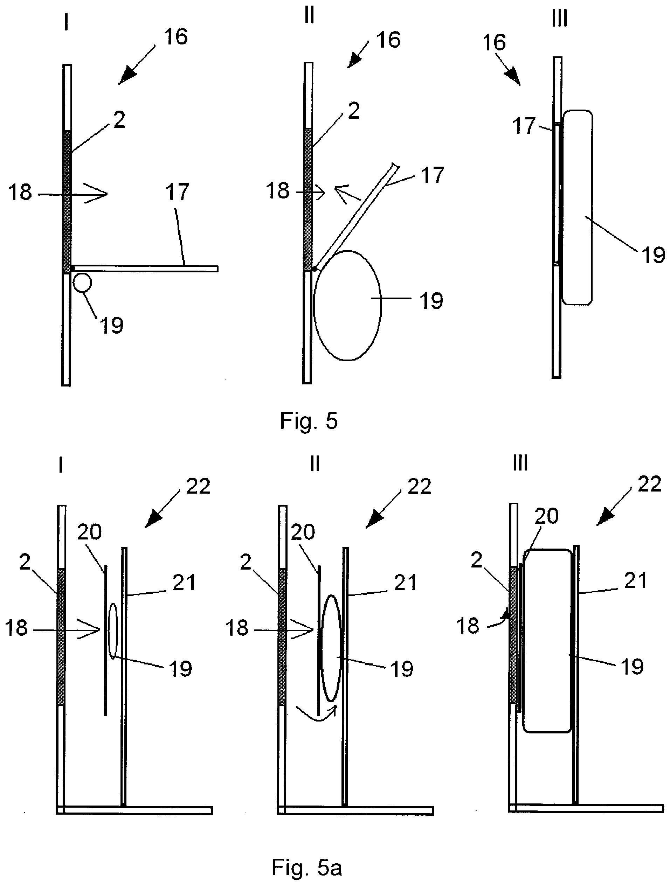

The inlet 2 of the urine sampling device 1 is provided with a feature that seals the chamber 3 after receiving the amount of urine that fills the chamber 3. The inlet 2 can be closed in the same way as described in the document WO 2008/131 904, i.e. were a swellable material closes the inlet 2. An alternative embodiment of the inlet closing can be seen in Fig. 5. The figure discloses a portion of the housing of the urine sampling device 1 comprising the inlet 2 wherein the inlet 2 is closable by means of a closing device 16 comprising a movable hatch 17. Urine enters asln a filling sequence I - III the hatch 17 moves from a fully open position I to a semi open position II to a closed position III. The movement of the hatch 17 is provided by a swelling material 19. The swelling material 19 can be any suitable material known to the person skilled in the art. The material 19 should have an ability to swell that does not interfere with the dry gel provided in the housing 4 for sampling. In Fig.5a an alternative closing device 22 is disclosed where a slot formed by a inner wall 21 is provided. On the side of the wall 21 facing the inlet 2, a film 20 is provided. Sandwiched between the film 20 and the wall 22 is a swellable material 19 positioned. As urine enters, see arrow 18, it will partly penetrate behind the film 20 and the swellable material 19 will swell an thereby the film 20 will approach and close the inlet 2, in the sequence I - III. Swelling occurs in sufficiently slow manner such that the inlet 2 is not closed before the urine sampling device 1 is filled with urine.

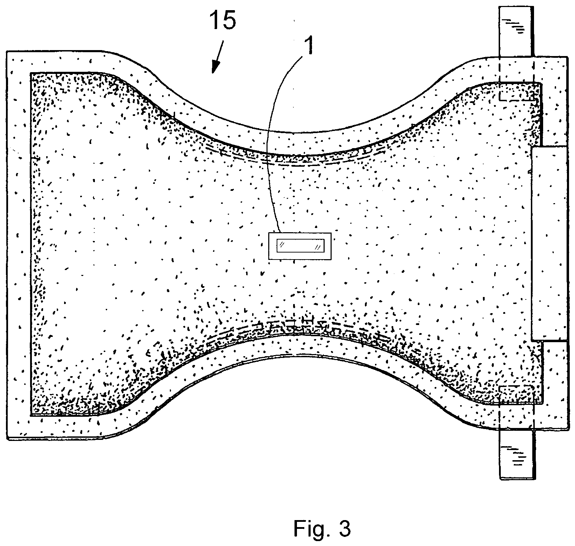

The housing 4 has attachment means 10 added to one of its outer walls. The attachment means 10 can be any suitable attachment means such as an adhesive, or a hook and loop device. The attachment means 10 should be able to attach the urine sampling device 1 to a sheet of an absorbent hygiene product 15. The outer wall in question need not be fully provided with the attachment means 10, a portion of the housing 4 can provided with the attachment means 10. In a preferred embodiment the attachment means 10 is suitable for attaching it to a top sheet, i.e. the body side, of an absorbent product 15 as seen in Fig. 3. Even though the drawing discloses the urine sampling device 1 as attached in a position which is external to the top sheet, the urine sampling device 1 could also be fitted under said top sheet. Another position that is possible is inside the absorbent layer of the hygiene product 15. Such a positioning is preferred when a higher comfort to the user is preferred.

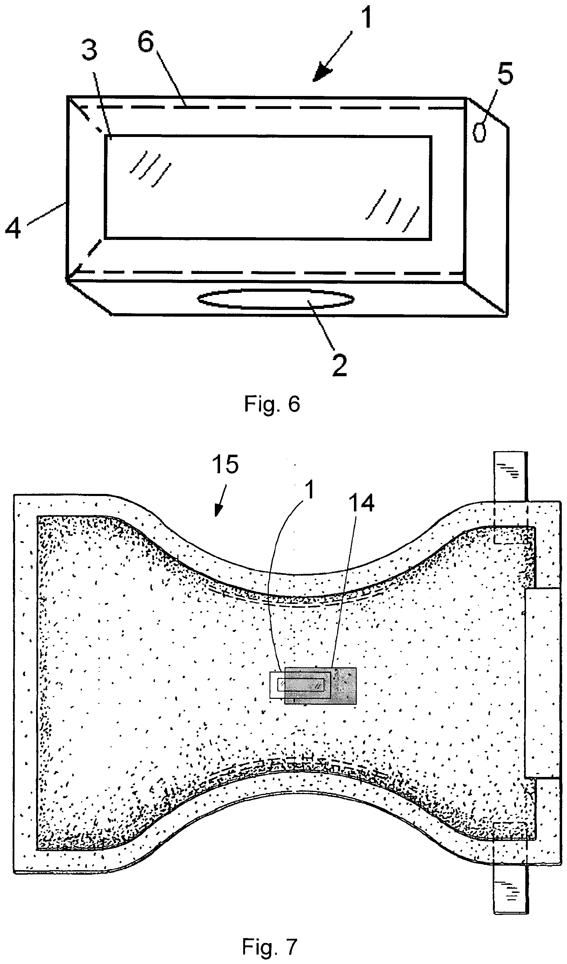

It is also possible to combine the urine sampling device 1 with an absorbent product 15 having a pocket 14 for residing the urine sampling device 1. See Fig. 7.

The urine sampling device 1 also can also be provided with a ventilation opening 5, for allowing air residing in the urine sampling device 1 to escape when the urine sampling device 1 receives the urine. The ventilation opening 5 need not be provided with a closing device 16 due to its size. However it could be provided with a closing device 16 as described above, or any other means for closing.

The urine sampling device 1 can also comprise a clear window 7. A urine sampling device 1 fitted with such a window 7 would be examinable before the gel is taken out, for example to see how well filled the urine sampling device 1 is, such that a new sample can be taken if the urine sampling device 1 is not properly filled. To further increase the ability of detecting if the urine sampling device is properly filled or not, an additive can be added to the urine sampling chamber which additive changes colour as urine enters the chamber. The colour change can be due to a colorant which is activateable by water solutions revealing a visual colour. An example of a suitable colorant is iron sulphate. A second possibility as to how a colour switch can be achieved is that if an acid is used as a preservative, such as boric acid, in combination with a pH indicator. Examples of suitable pH indicators are methyl red and cresol red.

The urine sampling device 1 could also be fitted with fractural impressions 6 as disclosed in Fig. 6. These impressions would make it easier to dismount the urine sampling device when the gel is to be taken out.

As mentioned above, inside the chamber 3 resides a dry gel that swells when subjected to urine. The gel preferably has inherent properties which are able to preserve a group of bacteria that has followed the urine into the urine sampling device 1, thus preventing substantive growth of the bacteria. This effect has been proven in examples 1 and 2 provided below. The gel can also be mixed with a suitable preservative such as Boric acid, or any other suitable preservative. The preservative provides an even more stabile system for preventing growth of the sampled bacteria. When applying a preservative together with a gel having preservative properties of its own, a surprising synergistic effect has been found that provides for an even more effective preservation effect. The gel can preferably be provided in dry form, for example as a powder or a film. A gel made of polyethyleneoxide (PEO) has the mentioned inherent preservative effect. The dry gel is a hydrophilic physically cross linked gel. This gel can consist of polysaccharides such as a CMC based gel. This is considered an advantage as the gel would become biodegradable. The dry gel can also consist of synthetic polymers, preferably hydrophilic polymers. This is advantageous as the polymers can be provided homogenously in composition from batch to batch.

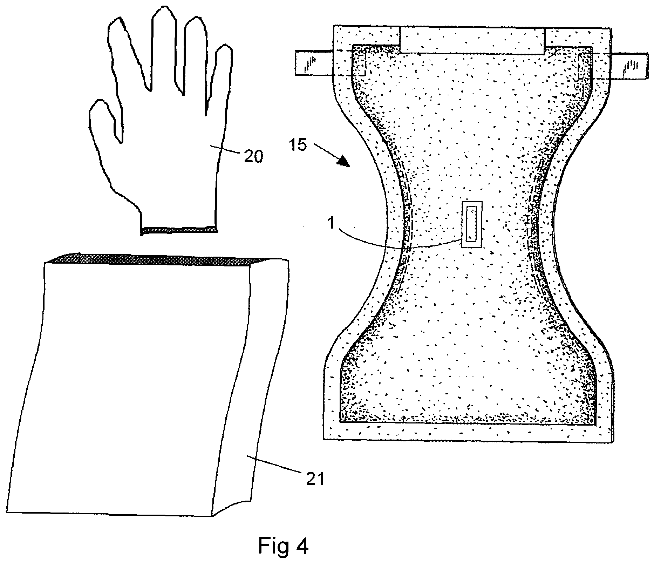

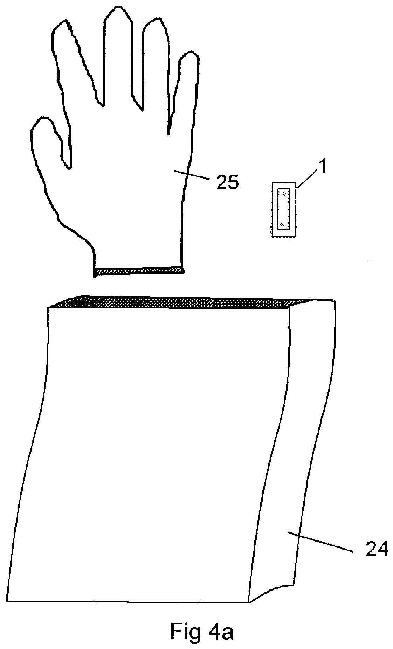

By providing the device in a urine sampling kit as can be seen in Fig 4a the urine sampling can be simplified. The device is provided with a container 24 for transport to an analysing site. There can also be provided a glove 25. And there can also be provided an absorbent product 15 provided with the urine sampling device 1 preinstalled, as seen in Fig. 4.

### Example 1: Testing the precision of the system

It is important that the amount bacteria can be correctly determined. The purpose of this test was to ensure that the determined number of bacteria using the urine sampling device does not differ more than acceptable from what is determined directly from a solution

In this test an overnight culture of an E.coli (CCUG 49263, NCTC 10538) was diluted in tenfold steps in Tryptic Soy Broth (TSB) and saline (1:9). Three of these dilutions were measured directly with traditional pour plate technique from the solution (TSA) and compared to the determined amounts when using the urine sampling device.

The urine sampling device was produced as follows: A 5% (w/w) water solution of polyethyleneoxide (PEO) from Scientific Polymer Products (Mw= 200 000g/mole) was produced by dispersing the powder in water and subsequently stirring the solution for at least 12 hrs. The solution was then transferred to a small aluminium mould (diameter 2.5 cm and depth 1.5 cm) and freezed at -80°C for at least 12 hrs. The aluminum mould was dismounted from the freezed solution before it was put in the freeze dryer (FD 3 from Heto Lab Equipment) until all water was sublimated. The freeze dried PEO was then cut in halves and one half was used in all experiments. When boric acid was used as a preservative it was added to the PEO solution before the freeze drying step.

0.2 ml of E.coli suspension was added to the dry PEO-gel. After some minutes the gel was put in 10 ml NaCl and dissolved (about 10 min). After that the PEO gel was totally dissolved, the amounts of bacteria were determined again with pour plate on TSA.

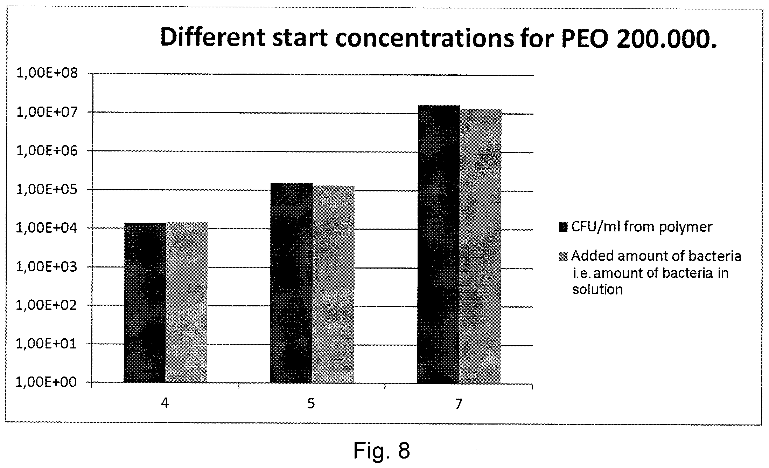

In Figure 8 the numbers determined directly from suspension are compared to the numbers determined when the suspension was trapped by the dry gel, which was then dissolved in saline and counted afterwards.

As can be seen in the Figure 8 almost the equal amount of bacteria is determined directly from suspension compared to suspension trapped in PEO gel.

### Example 2: Testing the stability of the system

The purpose of this test was to ensure that the system is stable and that time between urination and sampling can be at least 12 hours without changing the analyzed result more than +/- one log unit. The gel should preserve the trapped microorganisms without killing them in any significant amount. This can be achieved either in that the polymer can have a sufficient preservative effect in itself or this effect can be further enhanced by using an extra additive with preservative effect.

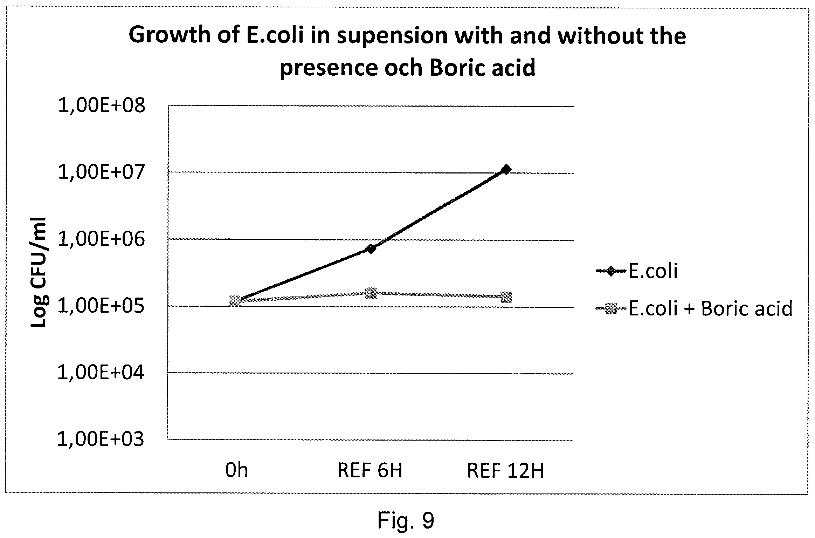

In a first study a suspension with TSB saline (1:9) was inoculated with 10⁵ CFU/ml of an uropathogenic E.coli and allowed to grow for 12 hours under studying. In the Figure 9 it can be seen that in the E.coli when in suspension grows 2 logs in the non-preserved system and in the system with preservative a stable system is achieved by the addition of 1,8 % (w/w) of Boric acid as preservative.

In the second experiment in of example 2, a dried gel with PEO (100 000g/mole) was poured with 200 µl of TSB saline (1:9) with an E.coli (10⁵ CFU/ml). The gel was taken out directly (after 5 minutes), after 6 hours and after 12 hours. The gel was then dissolved in 10 ml NaCl and analyzed with pour-plate cultivation on TSA.

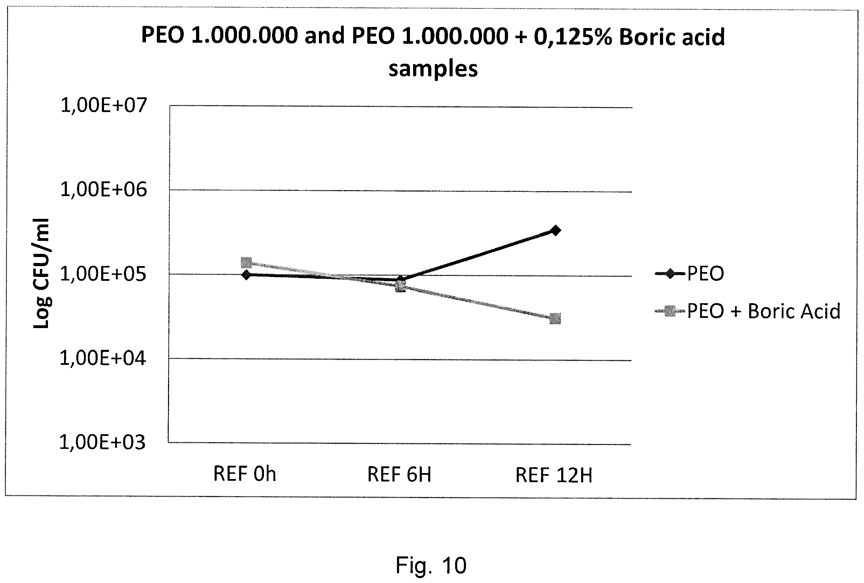

As can be seen in the Figure 10 the PEO in itself has a preservative effect. The measured increase of E.coli measured after 12 hours is 0,5 log units (compared to two log increase in Figure 9). With an addition of Boric acid (0,125 % (w/w)*) to the dried PEO gel a decrease of 0,5 log units was measured after 12 hours. A deviation of 0,5 log units after 12 hours may be acceptable for this measuring of the urine sampling device. And there is absolutely a potential for further adjustments to make a system that is very stable for at least 12 hours.

* The amount of boric acid is denoted as the amount added to the PEO solution and is a rough estimation of the amount necessary in the dry gel, such that the 200 µl of TSB saline (1:9) with E.coli that hits the urine sampling device will encounter 1,8 % (w/w) of boric acid.

## Claims

1. A urine sampling device (1) comprising:
a housing (4) comprising a urine sampling chamber (3),
the housing (4) of the urine sampling device (1) being arranged such that the urine sampling device (1) can be associated with a hygiene absorbent product (15), and
the urine sampling device (1) comprises an inlet (2) for said fluid,
**characterized in that** an outer surface of the device (1) comprises an attachment means (10), for attaching the urine sampling device (1) to the body side of the hygiene absorbent product (15), or the hygiene absorbent product (15) comprises a pocket (14) for incorporating the urine sampling device (1), and **in that** said chamber (3) comprises a dry gel that can be swelled by urine, wherein said gel is a physically cross linked hydrophilic gel, and is chosen from a group consisting of polysaccharides and synthetic hydrophilic polymers, and said hydrophilic polymer is polyethyleneoxide (PEO),and wherein said urine sampling device (1) possess the ability to preserve a bacteria content from a urine sampled in the urine sampling device (1), either by means of the properties of the dry gel in itself and/or by providing a preservative chosen from a group consisting of acids such as boric acid, and salts from these such as sodium borate or potassium borate, in the dry gel.

2. The urine sampling device (1) according to claim 1, wherein said preservative is present in an amount such that any bacteria present in a urine sample that has entered said chamber (3) can be held in said chamber (3) for a period of at least 12 hours, where growth of bacteria is less than one log unit and/or death of bacteria is less than one log unit.

3. The urine sampling device (1) according to claim 1, wherein said attachment means (10) is arranged such that it can detachably attach the urine sampling device (1) to said absorbent product (15).

4. The urine sampling device (1) according to claim 3, wherein said attachment means (10) is an adhesive, or a hook and loop arrangement.

5. The urine sampling device (1) according to any of the claims 1-4, wherein said chamber (3) comprises a selected amount of dry gel, in order to swell and hold a predetermined volume of urine in the chamber (3).

6. The urine sampling device (1) according to claim 5, wherein said volume is comprised in the interval 100 µl to 5000 µl preferably 150 µl to 500 µl.

7. The urine sampling device (1) according to any of the claims 1-6, further comprising a closing device (16) for closing said inlet (2) to seal the chamber (3) when said urine sampling device (1) has received a predetermined volume of urine.

8. The urine sampling device (1) according to any of the claims above, wherein the urine sampling device (1) is dismountable by means of fractural impressions (6) made in the housing (3), such that a urine sample absorbed by said gel, can be removed from said chamber (3), for further examination.

9. The urine sampling device (1) according to any of the claims above, wherein the housing (3) of the urine sampling device (1) comprises a ventilation opening (5) for ventilating air out of the urine sampling device (1) when it is filled with urine.

10. A hygiene absorbent product (15), **characterized in that** it comprises an associated urine sampling device (1) according to any of the claims 1 -9.

11. A urine examination kit, comprising a urine sampling device (1) according to any of the claims 1 - 9and a sample collection container (24) arranged to house the urine sampling device (1), for transport of said urine sampling device (1) for further examination.

12. The urine examination kit of claim 11, further comprising a glove (25) for preventing contamination of the urine sampling device (1) when removing it from the hygiene absorbent product (15).

## Patentansprüche

1. Urinprobennahmevorrichtung (1) mit:
einem Gehäuse (4) mit einer Urinprobennahmekammer (3),
wobei das Gehäuse (4) der Urinprobennahmevorrichtung (1) so angeordnet ist, dass die Urinprobennahmevorrichtung (1) mit einem saugfähigen Hygieneprodukt (15) verbunden werden kann und die Urinprobennahmevorrichtung (1) einen Einlass (2) für die Flüssigkeit aufweist,
**dadurch gekennzeichnet, dass** eine äußere Fläche der Vorrichtung (1) ein Anbringmittel (10) zum Anbringen der Urinprobennahmevorrichtung (1) an der Körperseite des saugfähigen Hygieneprodukts (15) aufweist oder das saugfähige Hygieneprodukt (15) eine Tasche (14) zum Aufnehmen der Urinprobennahmevorrichtung (1) aufweist, und dadurch, dass die Kammer (3) ein Trockengel aufweist, das durch Urin aufquellbar ist, wobei das Gel ein physisch vernetztes hydrophiles Gel ist und aus einer Gruppe ausgewählt ist, die aus Polysacchariden und synthetischen hydrophilen Polymeren besteht, das hydrophile Polymer Polyethylenoxid (PEO) ist und die Urinprobennahmevorrichtung (1) die Eigenschaft aufweist, einen Bakteriengehalt von entnommenem Urin entweder mittels der Eigenschaften des Trockengels selbst und/oder durch Bereitstellen eines Konservierungsmittels, das aus einer Gruppe ausgewählt ist, die aus Säuren, wie zum Beispiel Borsäure, und Salzen aus diesen, wie zum Beispiel Natriumborat oder Kaliumborat, besteht, in dem Trockengel in der Urinprobennahmevorrichtung (1) aufzubewahren.

2. Urinprobennahmevorrichtung (1) nach Anspruch 1, bei der das Konservierungsmittel in so einer Menge vorliegt, dass jegliche Bakterien, die in einer Urinprobe vorliegen, die in die Kammer (3) eingetreten ist, in der Kammer (3) über einen Zeitraum von mindestens zwölf Stunden gehalten werden kann, wobei die Vermehrung der Bakterien geringer ist als eine logarithmische Einheit und/oder der Tod der Bakterien geringer ist als eine logarithmische Einheit.

3. Urinprobennahmevorrichtung (1) nach Anspruch 1, bei der das Anbringmittel (10) so angeordnet ist, dass es die Urinprobennahmevorrichtung (1) an dem saugfähigen Produkt (15) abnehmbar anbringen kann.

4. Urinprobennahmevorrichtung (1) nach Anspruch 3, bei der das Anbringmittel (10) ein Haftmittel oder eine Haken- und Schlaufenanordnung ist.

5. Urinprobennahmevorrichtung (1) nach einem der Ansprüche 1-4, bei welcher die Kammer (3) eine gezielte Menge von Trockengel aufweist, um aufzuquellen und ein vorbestimmtes Urinvolumen in der Kammer (3) zu halten.

6. Urinprobennahmevorrichtung (1) nach Anspruch 5, bei der das Volumen sich in einem Intervall von 100 µl bis 5000 µl, bevorzugt 150 µl bis 500 µl, befindet.

7. Urinprobennahmevorrichtung (1) nach einem der Ansprüche 1-6, ferner mit einer Schließeinrichtung (16) zum Verschließen des Einlasses (2), um die Kammer (3) zu versiegeln, wenn die Urinprobennahmevorrichtung (1) ein vorbestimmtes Urinvolumen empfangen hat.

8. Urinprobennahmevorrichtung (1) nach einem der obigen Ansprüche, wobei die Urinprobennahmevorrichtung (1) mittels in dem Gehäuse (3) hergestellter Brecheindrücke (6) zerlegbar ist, sodass eine in dem Gel absorbierte Urinprobe für eine weitere Untersuchung aus der Kammer (3) entfernt werden kann.

9. Urinprobennahmevorrichtung (1) nach einem der obigen Ansprüche, bei der das Gehäuse (3) der Urinprobennahmevorrichtung (1) eine Lüftungsöffnung (5) zum Entlüften von Luft aus der Urinprobennahmevorrichtung (1) aufweist, wenn sie mit Urin gefüllt wird.

10. Saugfähiges Hygieneprodukt (15), **dadurch gekennzeichnet, dass** es eine zugehörige Urinprobennahmevorrichtung (1) nach einem der Ansprüche 1-9 aufweist.

11. Urinuntersuchungskit mit einer Urinprobennahmevorrichtung (1) nach einem der Ansprüche 1-9 und einem Probensammelbehälter (24), der eingerichtet ist, die Urinprobennahmevorrichtung (1) für einen Transport der Urinprobennahmevorrichtung (1) zwecks weiterer Untersuchung aufzunehmen.

12. Urinuntersuchungskit nach Anspruch 11, ferner mit einem Handschuh (25), um einer Kontamination der Urinprobennahmevorrichtung (1) vorzubeugen, wenn sie von dem saugfähigen Hygieneprodukt (15) entfernt wird.

## Revendications

1. Dispositif de prélèvement d'urine (1) comprenant :
un logement (4) comprenant une chambre de prélèvement d'urine (3),
le logement (4) du dispositif de prélèvement d'urine (1) étant agencé pour que le dispositif de prélèvement d'urine (1) puisse être associé à un produit absorbant hygiénique (15), et le dispositif de prélèvement d'urine (1) comprend une entrée (2) pour ledit fluide,
**caractérisé en ce qu'**une surface externe du dispositif (1) comprend un moyen d'attache (10), destiné à attacher le dispositif de prélèvement d'urine (1) au côté corps du produit absorbant hygiénique (15), ou le produit absorbant hygiénique (15) comprend une poche (14) destinée à incorporer le dispositif de prélèvement d'urine (1), et **en ce que** ladite chambre (3) comprend un gel sec qui peut être gonflé par l'urine, dans lequel ledit gel est un gel hydrophile physiquement réticulé, et est choisi dans le groupe consistant en les polysaccharides et des polymères hydrophiles synthétiques, et ledit polymère hydrophile est le polyoxyde d'éthylène (PEO), et dans lequel ledit dispositif de prélèvement d'urine (1) possède la capacité de préserver une teneur en bactéries d'une urine prélevée dans le dispositif de prélèvement d'urine (1), soit au moyen des propriétés du gel sec lui-même et/ou en fournissant un conservateur choisi dans le groupe consistant en des acides tels que l'acide borique, et des sels de ceux-ci tels que le borate de sodium ou le borate de potassium, dans le gel sec.

2. Dispositif de prélèvement d'urine (1) selon la revendication 1, dans lequel ledit conservateur est présent dans une quantité telle que toute bactérie présente dans un échantillon d'urine qui est entré dans ladite chambre (3) puisse être contenue dans ladite chambre (3) pendant une période d'au moins 12 heures, où la croissance de bactéries est inférieure à une unité log et/ou la mort de bactéries est inférieure à une unité log.

3. Dispositif de prélèvement d'urine (1) selon la revendication 1, dans lequel ledit moyen d'attache (10) est agencé pour qu'il puisse attacher de façon détachable le dispositif de prélèvement d'urine (1) audit produit absorbant (15).

4. Dispositif de prélèvement d'urine (1) selon la revendication 3, dans lequel ledit moyen d'attache (10) est un adhésif, ou un agencement de type fermeture autoagrippante.

5. Dispositif de prélèvement d'urine (1) selon l'une quelconque des revendications 1 à 4, dans lequel ladite chambre (3) comprend une quantité sélectionnée de gel sec, afin de gonfler et de contenir un volume prédéterminé d'urine dans la chambre (3).

6. Dispositif de prélèvement d'urine (1) selon la revendication 5, dans lequel ledit volume est compris dans l'intervalle de 100 µL à 5 000 µL, de préférence de 150 µL à 500 µL.

7. Dispositif de prélèvement d'urine (1) selon l'une quelconque des revendications 1 à 6, comprenant en outre un dispositif de fermeture (16) destiné à fermer ladite entrée (2) pour obturer la chambre (3) lorsque ledit dispositif de prélèvement d'urine (1) a reçu un volume d'urine prédéterminé.

8. Dispositif de prélèvement d'urine (1) selon l'une quelconque des revendications précédentes, dans lequel le dispositif de prélèvement d'urine (1) peut être démonté au moyen d'empreintes fracturées (6) pratiquées dans le logement (3), de sorte qu'un échantillon d'urine absorbé par ledit gel puisse être enlevé de ladite chambre (3), pour un examen approfondi.

9. Dispositif de prélèvement d'urine (1) selon l'une quelconque des revendications précédentes, dans lequel le logement (3) du dispositif de prélèvement d'urine (1) comprend une ouverture de ventilation (5) destinée à ventiler l'air hors du dispositif de prélèvement d'urine (1) lorsqu'il est rempli d'urine.

10. Produit absorbant hygiénique (15), **caractérisé en ce qu'**il comprend un dispositif de prélèvement d'urine (1) associé selon l'une quelconque des revendications 1 à 9.

11. Kit d'examen d'urine, comprenant un dispositif de prélèvement d'urine (1) selon l'une quelconque des revendications 1 à 9 et un récipient de collecte d'échantillon (24) agencé pour loger le dispositif de prélèvement d'urine (1), pour un transport dudit dispositif de prélèvement d'urine (1) pour un examen approfondi.

12. Kit d'examen d'urine selon la revendication 11, comprenant en outre un gant (25) destiné à empêcher une contamination du dispositif de prélèvement d'urine (1) lorsqu'il est enlevé du produit absorbant hygiénique (15).
